# EUROPEAN PATENT APPLICATION

(11) **EP 1 362 616 A1**
(43) Date of publication of application: **19.11.2003**
(21) Application number: 02076894.1
(22) Date of filing: 15.05.2002
(51) Int. Cl.: A61N 5/10

(54) **Method and device for providing a radiation therapy treatment planning solution**

(71) Applicant: Nucletron B.V., 3905 TH Veenendaal (NL)
(72) Inventor: Kindlein, Johann, 46049 Oberhausen (DE); Scholte, Rudolf Leonard Josef, 3818 GP Amersfoort (NL); Adam, Mark Alexander, 1072 MK Amsterdam (NL); Nuijten, Arnoldus Gerardus, 5626 GG Eindhoven (NL)
(74) Representative: Valkonet, Rutger

(57) **Abstract**

The invention relates to a method for providing a radiation therapy treatment planning solution for a radiation therapy treatment of cancerous tumours in an animal body, wherein said radiation therapy treatment planning solution is selected from a plurality of radiation therapy treatment plans.

The invention furthermore relates to a device for providing a radiation therapy treatment planning solution for a radiation therapy treatment of cancerous tumours in an animal body, wherein said radiation therapy treatment planning solution is selected from a plurality of radiation therapy treatment plans.

## Description

The invention relates to a method for providing a radiation therapy treatment planning solution for a radiation therapy treatment of tumours in an animal body, wherein said radiation therapy treatment planning solution is selected from a plurality of radiation therapy treatment plans.

The invention furthermore relates to a device for providing a radiation therapy treatment planning solution for a radiation therapy treatment of tumours in an animal body, wherein said radiation therapy treatment planning solution is selected from a plurality of radiation therapy treatment plans.

The determination of the optimum conditions for an accurate and precise radiation therapy plan continues to be a major concern in radiation therapy. For performing the treatment planning specialist with a large experience are required, apart from additional expensive hightech equipment, such as CT- or MRI-scanning systems.

For the treatment of tumours, e.g. cancerous tumours, in an animal body using high energy radiation, such as high energy radiation delivered by linear accelerators or other types of radiation emitting devices all kinds of radiation therapy treatment planning parameters are required for planning an adequate radiation therapy treatment solution for the radiation therapy treatment of the tumour.

At present images or slices of the animal body near the position and location of the tumour to be treated, are prepared using known CT- and or MRI- scanning systems for obtaining parameters relating to the structure of the patient's body, the exact location of the tumour, as well as its size inside the patients body. These parameters together with patient specific parameters, such as the gender, age and dimensions of the patient are entered into a doses planning software program loaded in a computer system. Furthermore specific characteristic of the radiation emitting device used by the hospital are entered into said doses planning software program and used for planning a radiation therapy treatment solution for the intended radiation therapy treatment of the tumour.

During the planning procedure different radiation therapy treatment solutions are planned and simulated on said computer system for experimenting purposes. With these computer simulations hypothetical radiation therapy treatments with different radiation characteristics, such as radiation doses, radiation field characteristic and distributions as emitted by the used radiation emitting device are examined by the radiation therapy specialist until the best radiation therapy treatment planning solution has been found for the actual radiation therapy treatment of the patient. The use of sophisticated doses planning software programs on computer systems aims to preplan and obtain the most effective radiation treatment of the tumour with a minimum of risk to the patient and or the radiation therapy specialist in view of overexposure to high energy radiation.

However it will be clear that doses treatment planning is a very time-consuming process, whereas for young medical physicians the decision whether the proposed radiation therapy treatment plan generated by the doses planning software program can be applied for the radiation therapy treatment of a patient is a big challenge and a rather great burden to the responsibility.

The invention aims to provide a helpful tool for a radiotherapy institution, such as a hospital for obtaining a radiation therapy planning solution best fitted to a medical case without the use of complicated doses planning software and time-consuming computer simulations.

According to the invention the method is characterized in that storing a plurality of radiation therapy treatment plans on a machine readable medium classifying said plurality of radiation therapy treatment plans on said machine readable medium in function of a plurality of radiation therapy treatment planning parameters and accessing said machine readable medium for obtaining at least one specific radiation therapy treatment planning solution for a specific radiation therapy treatment.

In brief the invention is directed in comparing a specific medical case of a patient for which a radiation therapy treatment solution has to be prepared with a collection of other medical cases previously conducted (read: patients) and presenting/proposing a radiation therapy treatment planning solution relating to a previous medical case to the radiation therapy specialist as a possible treatment solution of that specific medical case.

More particularly the method according to the invention is characterized by the step of obtaining said specific radiation therapy treatment planning solution in response to a query configured and conducted on said plurality of radiation therapy treatment planning parameters by a specific user, wherein said query is configured using user defined radiation therapy treatment planning parameters. The user or the radiotherapy treatment specialist who is seeking for a specific radiotherapy treatment planning solution has to enter radiation therapy treatment planning parameters relating to his specific medical case after which the radiotherapy treatment planning solution that fits his specific medical case best is presented to the user.

In fact the method according to the invention matches the query of the user defined radiation therapy treatment planning parameters with said plurality of radiation therapy treatment planning parameters stored on said machine readable medium.

In another more sophisticated embodiment of the invention the method can use the query of user defined radiation therapy treatment planning parameters to plan a specific radiation therapy treatment and to present that solution to the user. Hence the user or radiotherapy treatment specialist do not need to perform the time consuming treatment planning simulations, but according to the method of the invention a radiotherapy treatment planning solution is prepared and presented using said plurality of radiation therapy treatment planning parameters stored on said machine readable medium.

In order to avoid misuse of the radiotherapy treatment method according to the invention, an authorisation status of said user can be verified prior to the step of accessing said machine readable medium by the user or prior to the step of presenting said specific radiation therapy treatment planning parameters or said specific radiation therapy treatment planning solution to the user.

The user can use the specific radiation therapy treatment planning solution or corresponding specific radiation therapy treatment planning parameters for preparing and preforming the radiotherapy treatment on the user's patient.

According to a specific embodiment of the invention a new radiation therapy treatment plan can be added and classified in function of its corresponding radiation therapy treatment planning parameters to said plurality of radiation therapy treatment plans and stored on said machine readable medium. This allows the system to be updated with a new "case", which can be used in the future as a new radio therapy treatment planning solution.

Preferably according to the invention said new radiation therapy treatment plan has to be verified and tested in order to prevent that an "unfounded", in medical terms "unsuited" radiation therapy treatment plan is added and stored on said machine readable medium.

The invention also relates to a device for providing a radiation therapy treatment planning solution for a radiation therapy treatment of tumours in an animal body, wherein said radiation therapy treatment planning solution is selected from a plurality of radiation therapy treatment plans according to the method according to the invention, at least comprising processing and memory means.

According to the invention the device for providing a radiation therapy treatment planning solution is characterized in that a plurality of radiation therapy treatment plans are stored in said memory means in function of a plurality of radiation therapy treatment planning parameters, wherein said device is capable of setting up an information communication link with at least one remote computer terminal of an user, allowing said user via said communication link access to said device for obtaining at least one specific radiation therapy treatment planning solution from said plurality of radiation therapy treatment plans for a specific radiation therapy treatment.

With such a device according to the invention a plurality of radiation therapy treatment plans are collected and managed at a location which is made accessible for one or more users, which user can select at least one of those radiation therapy treatment plans as a possible solution for a specific medical case.

Said user can configure a query on said plurality of radiation therapy treatment plans stored in said device, which device further more according to the invention is characterized in that means are present for conducting said query on said plurality of radiation therapy treatment planning parameters, for selecting said specific radiation therapy treatment planning solution from said plurality of radiation therapy treatment plans and for transmitting said specific radiation therapy treatment planning solution via said communication link towards said user.

In a specific embodiment the specific parties using the method and device according to the invention may communicate with each other via a computer network, for example the internet network.

The invention shall now be described with the aid of the accompanying drawing, which show:

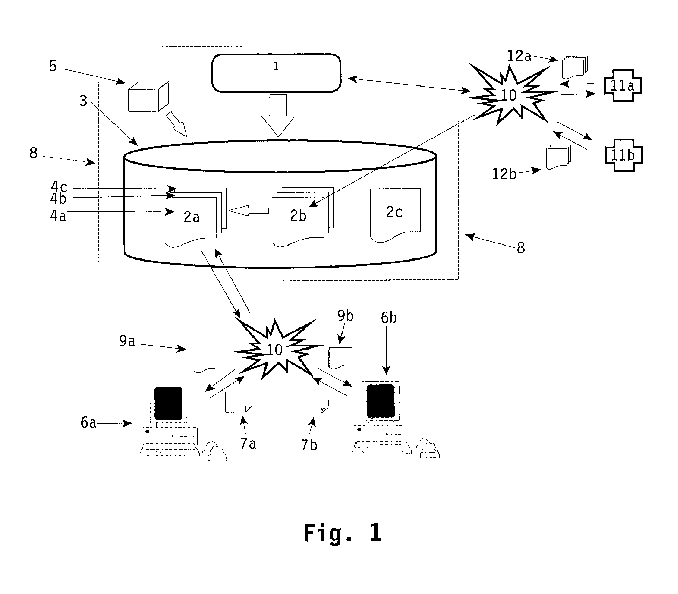

Figure 1 a schematic illustration of an embodiment of the method and device according to the invention.

Figure 1 shows an example of a method and device according to the invention for providing a radiation therapy treatment planning solution for a radiation therapy treatment of tumours in an animal body. At present a radiation therapy treatment planning solution for a radiation therapy treatment is preplanned using suitable doses planning software, such as a doses planning software system developed by Nucletron B.V. and marked under the trade name PLATO™. When using such a radiation treatment planning software system medical personal can prepare and conduct the intended radiotherapy treatment on a simulated patients body, which simulated patient's body is stored into the computer system, which is executing the doses planning software.

Although such doses planning software systems are very useful for performing simulations of radiation therapy treatments, the use and implementation of such doses planning software requires not only specific designed computer systems, but moreover highly qualified medical personal, which specialists require a large clinical experience. Moreover radiation therapy treatment planning can be a very time-consuming process and for less experienced medical personnel it is difficult to decide whether the radiotherapy treatment plan as proposed by the radiotherapy treatment planning software can be applied for the actual radiotherapy treatment of the patient.

In figure 1 an alternative is proposed for the present radiotherapy treatment planning software. Primarily the method and device according to the invention for providing a radiation therapy treatment planning solution for a radiation therapy treatment of tumours in a body consist of a machine readable medium 3 which is for example contained in a computer system. On said machine readable medium which can be a hard disk or other suitable storage means one or more database configurations 2a, 2b and 2c are incorporated. Especially database 2a is used for storing a plurality of existing radiation therapy treatment plans. According to an important aspect of the invention said plurality of existing radiation therapy treatment plans are stored on said machine readable medium 3 in function of a plurality of radiation therapy treatment planning parameters, in figure 1 depicted by means of the reference numerals 4a, 4b and 4c.

Said plurality of radiation therapy treatment planning parameters 4a-4c which are used for classifying said plurality of radiation therapy treatment plans may relate to information, which information parameters are necessary for performing said existing radiation therapy treatment plan. For example the radiation therapy treatment planning parameters may relate to the gender, the age, the dimensions and the weight of one or more known patients (reference numeral 4a) or the type, dimensions and the location of a known tumour or tumours in said known patients body (reference numeral 4b) and to the radiation characteristics, such as radiation doses, radiation field characteristic and distributions from known radiation emitting devices, which are used for performing said radiation therapy treatments (reference numeral 4c).

It will be clear that the information parameters concerning the patients are stored in an anonymous manner on said database 2a on said machine readable medium 3.

The computer system 3 containing the several databases 2a, 2b and 2c stored on said machine readable medium is managed and controlled by a so called supervisor authority 1, which supervising authority 1 or institution maintains and updates the device according to the invention.

The computer system 3 containing the machine readable medium with the databases 2a, 2b and 2c may also include a radiation therapy treatment planning software system 5, which can be incorporated within the computer system 3 or it may incorporated on a separate computer system which is capable of communicating with said computer system 3.

Said machine readable medium 3 can be accessed for obtaining at least one specific radiation therapy treatment planning solution for a specific radiation therapy treatment. More in particularly a specific user (depicted by reference numerals 6a or 6b) may obtain access to said machine readable medium 3 via a communication link 10 using a remote computer (6a or 6b). Preferably said communication link 10 can be a telephone communication link or a computer network, for example the internet.

For obtaining at least one specific radiation therapy treatment planning solution from said plurality of radiation therapy treatment plans stored on said machine readable medium 3 a query is configured and conducted on said plurality of radiation therapy treatment planning parameters 4a-4c by said specific user 6a or 6b.

In Figure 1 said query is depicted with reference numeral 7a or 7b, which query is communicated via said communication link 10 to the computer system 3. Using specific algorithms said computer system 3 conducts said query 7a or 7b on said plurality of radiation therapy treatment planning parameters 4a-4c which are used for classifying said plurality of radiation therapy treatment plans in the database 2a.

More in particularly said computer system 3 is designed for matching the query of user defined radiation therapy treatment planning parameters 7a (7b) with said plurality of radiation therapy treatment planning parameters 4a-4c on said machine readable medium 3 using specific algorithms.

Once the computer system 3 has obtained at least one specific radiation therapy treatment planning solution from said plurality of radiation therapy treatment plans due to the matching of the query 7a (7b) presented by the user 6a (6b), said specific radiation therapy treatment planning solution is presented via said communication link 10 to the specific user 6a (6b). This step is in figure 1 depicted by reference numeral 9a (9b).

Said specific radiation therapy treatment planning solution obtained from the database 2a and presented via communication link 10 to the specific user 6a or 6b can be used by said specific user 6a or 6b for performing a specific radiation therapy treatment on a specific patient of the user 6a or 6b. For example said specific radiation therapy treatment planning solution 9a or 9b can be used by the specific user 6a or 6b for setting up a radiation therapy treatment for said specific patient.

It will be clear that the query sent to the computer system 3 by the specific user 6a or 6b comprises user defined radiation therapy treatment planning parameters, which parameters define information concerning the gender, the age, dimensions and the weight of a users specific patient, which patient is to be treated by the specific user 6a or 6b. The specific user 6a or 6b can be a hospital or a doctor who whishes to perform a radiation therapy treatment on said patients body. Moreover said user defined radiation therapy treatment planning parameters may concern the type, the dimensions and the location of the tumour or tumours to be treated in said user specific patients body, as well as radiation characteristics, such as radiation doses, radiation field characteristics and distributions from the radiation emitting device, which is used by said user 6a or 6b for performing radiation therapy treatments.

With the method and device according to the invention a specific user 6a or 6b (being an hospital or medical centre or a doctor) can obtain in a rather quick and easy manner a possible radiation therapy treatment planning solution, which can be used for performing a radiation therapy treatment on said user specific patient. Hence the use of complicated and expensive radiotherapy planning software, which not only require specific technical and clinical expertise of the user 6a or 6b but moreover requires time-consuming computations and simulations of possible treatment planning solutions are herewith avoided.

The access of the users 6a and 6b to the machine readable medium 3 for obtaining a possible radiation therapy treatment planning solution in response to a query 7a or 7b may be subject to a verification step for verifying an authorisation status of said specific user 6a or 6b. The method and device for providing a radiation therapy treatment planning solution according to the invention is subscription-based. This means that users 6a, 6b, etc. have to obtain a subscription from the supervising institution 1 in order to obtain access to the computer system 3 for obtaining a specific radiation therapy treatment planning solution.

Preferably, prior to the access of the user to said machine readable medium the system performs an authorisation status check in order to determine and to verify whether or not the user 6a or 6b has a valid subscription and is authorised to access the database 2a for querying the plurality of radiation therapy treatment plans. The authorisation and verification step of the subscription status of each user 6a or 6b is in Figure 1 depicted by the dashed line 8 surrounding the supervising institution 1, the computer system 3 containing the databases 2a, 2b and 2c as well as the radiation therapy doses planning software system 5. In case of an negative authorisation status check access to the database 2a is denied by the computer system 3.

Said security and authorisation check can be e.g. a computer software related firewall.

Another aspect of the method and device according to the invention concerns the possibility of inputting the query 7a or 7b containing the user defined radiation therapy treatment planning parameters into said radiotherapy planning software program 5 which program 5 uses said user defined parameters for creating a specific radiation therapy treatment planning solution especially designed according to said user defined radiation therapy treatment planning parameters. Hence each user 6a or 6b may use a remote radiotherapy planning software system for obtaining a radiation therapy treatment plan especially suited and directed to the medical case of the user specific patient.

Another aspect of the method and device according to the invention concerns adding and classifying a new radiation therapy treatment plan in function of its corresponding radiation therapy treatment planning parameters to said plurality of radiation therapy treatment plans stored in said database 2a on said machine readable medium 3. Preferably said new radiation therapy treatment plan 12a (or 12b) may be presented by specific medical centres 11a (11b) via a communication link 10, for example the internet. Preferably said new radiation therapy treatment plan 12a or 12b is verified and tested by said medical centres 11a, 11b, which centres are selected by the supervising institution 1. which manages the device according to the invention.

However said new radiation therapy treatment plans 12a, 12b can also be verified and tested by the supervising institution 1 itself, for example by using the radiotherapy doses planning software system 5. For the purpose of verifying and testing said new radiation therapy treatment plans 12a, 12b said new plans are preferably stored on a separate database 2b, which can not be accessed by any of the users 6a, 6b who have a subscription to the computer system 3.

Once the new radiation therapy treatment plans 12a, 12b, now stored in the database 2b, are approved by the supervising institution 1 or the specific medical centres 11a, 11b, said new radiation therapy treatment plans 12a, 12b are added to the database 2a. Once added, they can now serve as a possible solution for a users specific radiation therapy treatment plan of one of the users 6a, 6b provided that the query configured and inputted in the database 2a by said specific user 6a or 6b matches said new radiation therapy treatment plan 12a or 12b.

The communication between the users 6a, 6b and the specific medical centres 11a, 11b via the communication link 10 with the computer system 3 can be established in any suitable form for example using the well known mark-up language known as HTML presently used on the internet. However also other mark-up languages or programming languages, such as JAVA, PERL, Javascript, etc. can be used for a proper communication between the several parties involved and for presenting the necessary information on the remote computer terminals of the specific user 6a, 6b and the specific medical centres 11a, 11b.

The database 2c is used for storing all kinds of information which are used for a proper operation of the method and device according to the invention. More in particularly said database 2c can be used for storing subscription information relating to the users 6a, 6b and the specific medical centres 11a, 11b, e.g. contracts between the supervising institution 1 and the specific medical centres 11a, 11b, as well passwords etc for performing the authorisation status check of each user 6a, 6b prior to accessing the database 2a. The database 2c can also be used for invoicing the several users 6a, 6b etc.

## Claims

1. Method for providing a radiation therapy treatment planning solution for a radiation therapy treatment of tumours in an animal body, wherein said radiation therapy treatment planning solution is selected from a plurality of radiation therapy treatment plans, **characterized in that** the method comprises at least the steps of:
storing a plurality of radiation therapy treatment plans on a machine readable medium;
classifying said plurality of radiation therapy treatment plans on said machine readable medium in function of a plurality of radiation therapy treatment planning parameters; and
accessing said machine readable medium for obtaining at least one specific radiation therapy treatment planning solution for a specific radiation therapy treatment.

2. Method according to claim 1, further **characterized by** the step of obtaining said specific radiation therapy treatment planning solution in response to a query configured and conducted on said plurality of radiation therapy treatment planning parameters by a specific user.

3. Method according to claim 2, further **characterized by** configuring said query using user defined radiation therapy treatment planning parameters.

4. Method according to claim 3, further **characterized by** the step of matching the query of user defined radiation therapy treatment planning parameters with said plurality of radiation therapy treatment planning parameters on said machine readable medium.

5. Method according to claim 3, further **characterized by** the step of planning a specific radiation therapy treatment based on said query of user defined radiation therapy treatment planning parameters using said plurality of radiation therapy treatment planning parameters.

6. Method according to anyone to the claims 2-5, further **characterized by** the step of presenting said specific radiation therapy treatment planning parameters or said specific radiation therapy treatment planning solution to the user.

7. Method according to anyone of the preceding claims, further **characterized by** the step of verifying an authorisation status of said user prior to the step of accessing said machine readable medium by the user or prior to the step presenting said specific radiation therapy treatment planning parameters or said specific radiation therapy treatment planning solution to the user.

8. Method according to anyone of the preceding claims, further **characterized by** the step of adding and classifying a new radiation therapy treatment plan in function of its corresponding radiation therapy treatment planning parameters to said plurality of radiation therapy treatment plans stored on said machine readable medium.

9. Method according to claim 8, further **characterized by** the step of verifying and testing said new radiation therapy treatment plan prior to the step of adding said new plan on said machine readable medium.

10. Method according to anyone of the preceding claims, **characterized in that** said plurality of radiation therapy treatment planning parameters comprises information concerning:
the gender, the ages, the dimensions and the weights of one or more known patients stored in an anonymous manner,
the type, the dimensions and the location of a known tumour or tumours in a known patient's body, as well as
radiation characteristics, such as radiation doses, radiation field characteristics and distributions from known radiation emitting devices for performing radiation therapy treatments.

11. Method according to anyone of the preceding claims, **characterized in that** said user defined radiation therapy treatment planning parameters comprises information concerning:
the gender, the age, the dimensions and the weight of a user specific patient,
the type, the dimensions and the location of the known tumour or tumours in said user specific patient's body, as well as
radiation characteristics, such as radiation doses, radiation field characteristics and distributions from the radiation emitting device used by said user.

12. Method according to claim 8, **characterized in that** said added new radiation therapy treatment plan to said plurality of radiation therapy treatment plans stored on said machine readable medium are presented by third parties or users selected by the managing institution of said machine readable medium.

13. Method according to claim 12, **characterized in** storing a new radiation therapy treatment plan presented by a third party and a new radiation therapy treatment plan presented by an user on different locations on said machine readable medium.

14. Device for providing a radiation therapy treatment planning solution for a radiation therapy treatment of tumours in an animal body, wherein said radiation therapy treatment planning solution is selected from a plurality of radiation therapy treatment plans according to one or more of the above method claims, at least comprising
processing and memory means,
**characterized in that**,
a plurality of radiation therapy treatment plans are stored in said memory means in function of a plurality of radiation therapy treatment planning parameters, wherein said device is capable of
setting up an information communication link with at least one remote computer terminal of an user,
allowing said user via said communication link access to said device for obtaining at least one specific radiation therapy treatment planning solution from said plurality of radiation therapy treatment plans for a specific radiation therapy treatment.

15. Device according to claim 14 **characterized in that**, said device is designed to receive a query by a specific user.

16. Device according to claim 14 or 15, **characterized in that**, means are present for conducting said query on said plurality of radiation therapy treatment planning parameters, for selecting said specific radiation therapy treatment planning solution from said plurality of radiation therapy treatment plans and for transmitting said specific radiation therapy treatment planning solution via said communication link towards said user.
